# EUROPEAN PATENT APPLICATION

(11) **EP 4 483 899 A1**
(43) Date of publication of application: **01.01.2025**
(21) Application number: 23760086.1
(22) Date of filing: 24.02.2023
(51) Int. Cl.: A61K 39/395, A61K 31/17, A61K 31/519, A61K 38/21, A61K 45/00, A61P 35/00, A61P 35/02, A61P 43/00, C07K 16/46, C07K 16/18

(54) **MEDICINE COMPRISING COMBINATION OF ANTI-MUTANT-CALR ANTIBODY AND ANOTHER DRUG**

(30) Priority: 25.02.2022 JP 2022027751
(71) Applicant: Juntendo Educational Foundation, Tokyo 113-8421 (JP); Meiji Seika Pharma Co., Ltd., Tokyo 104-8002 (JP)
(72) Inventor: KOMATSU, Norio, Tokyo 113-8421 (JP); ISHIDA, Yoji, Tokyo 104-8002 (JP); ARAKI, Marito, Tokyo 104-8002 (JP); TSUCHIYA, Toshiyuki, Tokyo 104-8002 (JP); CHIKADA, Tsubasa, Tokyo 104-8002 (JP); IMAI, Misa, Tokyo 104-8002 (JP); FUKASAWA, Hiroshi, Tokyo 104-8002 (JP)
(74) Representative: Plougmann Vingtoft a/s
(86) International application number: PCT/JP2023/006642
(87) International publication number: WO 2023/163087

(57) **Abstract**

Provided is a pharmaceutical which exhibits sufficient anticancer effects by using an antibody which binds to a mutant CALR protein and another anticancer agent in combination. Provided is a pharmaceutical for preventing and/or treating a cancer, comprising (A) an antibody which specifically binds to a mutant calreticulin protein or a functional fragment thereof and (B) at least one drug selected from the group consisting of an alkylating agent, a platinum preparation, an antimetabolite, a ribonucleotide reductase inhibitor, a nucleotide analog, a topoisomerase inhibitor, a microtubule polymerization inhibitor, an antitumor antibiotic, an interferon, a cytokine preparation, a molecular targeting drug, a nucleic acid synthesis inhibitor, a JAK inhibitor, and a cancer immunotherapeutic agent in combination.

## Description

### Technical Field

The present invention relates to a pharmaceutical comprising an antibody having specificity for a mutant CALR protein or a functional fragment thereof and another drug in combination.

### Background Art

In a part of patients with Philadelphia-negative myeloproliferative neoplasms (MPNs), nucleotide deletion or insertion is found in exon 9 of the calreticulin (CALR) gene (Non Patent Literatures 1 and 2). It has been already revealed that the mutant CALR protein produced by a CALR mutated gene has oncogenicity independently causing a myeloproliferative neoplasm (MPN) by constitutively activating a thrombopoietin (TPO) receptor (Non Patent Literatures 3 to 6).

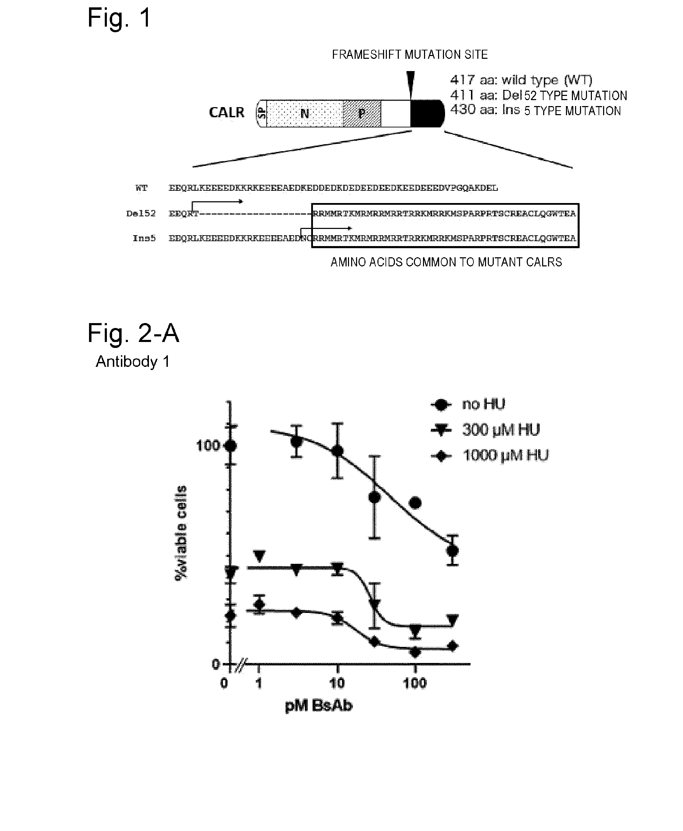

The CALR gene mutation found in MPN patients is a frameshift mutation which is always localized in the last exon, a sequence not present in the wild type is present at the C-terminus of the mutant CALR protein, and particularly, 44 amino acids at the C-terminus are common to almost all mutant CALR proteins. As an example thereof, Figure 1 shows a comparison of the C-terminal sequences of 52-nucleotide deletion type (Del 52), which is the most frequent mutation among CALR gene mutations found in MPN patients, and 5-nucleotide insertion type (Ins 5), which is the next most frequent CALR mutation, with the corresponding region of the wild-type CALR protein.

Since the mutant CALR protein causing an MPN is expressed in tumor cells, it has been suggested that a sequence specific to the mutant CALR protein caused by the frameshift mutation may become a diagnostic marker or a therapeutic target as a neoantigen (Patent Literatures 1 and 2).

Further, as a result of functional analysis of mutant CALR proteins, it has been revealed that, in addition to those represented by Del 52 and Ins 5, there are cleaved mutant CALR proteins, which have cleavages in sequences specific to the mutant CALR proteins, and in which most of the sequences inferred as neoantigens have been lost. When an antibody which specifically recognizes a very short amino acid sequence located at the N-terminal side of the cleavage site in the neoantigen and an antibody which binds to an amino acid sequence at the C-terminal side of the cleavage site are made, it has been revealed that an MPN therapeutic effect is obtained. Furthermore, an anti-CALR/CD3 bispecific antibody had been obtained as a more effective therapeutic agent by taking advantage of the properties of the antibody capable of specifically binding to each of two antigens for the purpose of enhancing antitumor activity.

On the other hand, in the field of low-molecular weight anticancer drugs, alkylating agents, platinum preparations, antimetabolites, ribonucleotide reductase inhibitors, nucleotide analogs, topoisomerase inhibitors, microtubule polymerization inhibitors, Bcl-2 inhibitors, antitumor antibiotics, interferons, cytokine preparations, molecular targeting drugs, nucleic acid synthesis inhibitors, JAK inhibitors, and cancer immunotherapeutic agents have been reported, but none of them has been satisfactory in terms of safety issues such as myelosuppression and hematotoxicity, and effectiveness.

### Citation List

### Patent Literature

Patent Literature 1: WO 2015/036599
Patent Literature 2: WO 2016/087514
Patent Literature 3: WO 1999/054440
Patent Literature 4: WO 2019/178362
Patent Literature 5: WO 2020/175689

### Non Patent Literature

Non Patent Literature 1: Klampfl T, Gisslinger H, Harutyunyan AS, et al. Somatic mutations of calreticulin in myeloproliferative neoplasms. The New England journal of medicine. 2013; 369: 2379-90.
Non Patent Literature 2: Nangalia J, Massie CE, Baxter EJ, et al. Somatic CALR mutations in myeloproliferative neoplasms with nonmutated JAK2. The New England journal of medicine. 2013; 369: 2391-405.
Non Patent Literature 3: Araki M, Yang Y, Masubuchi N, et al. Activation of the thrombopoietin receptor by mutant calreticulin in CALR-mutant myeloproliferative neoplasms. Blood. 2016; 127: 1307-16.
Non Patent Literature 4: Elf S, Abdelfattah NS, Chen E, et al. Mutant Calreticulin Requires Both Its Mutant C-terminus and the Thrombopoietin Receptor for Oncogenic Transformation. Cancer Discov. 2016; 6: 368-81.
Non Patent Literature 5: Marty C, Pecquet C, Nivarthi H, et al. Calreticulin mutants in mice induce an MPL-dependent thrombocytosis with frequent progression to myelofibrosis. Blood. 2016; 127: 1317-24.
Non Patent Literature 6: Vainchenker W, Kralovics R. Genetic basis and molecular pathophysiology of classical myeloproliferative neoplasms. Blood. 2017; 129: 667-79.

### Summary of Invention

### Technical Problem

The problem of the present invention is to provide a pharmaceutical which exhibits sufficient antitumor effects by using an antibody which binds to a mutant CALR protein and another anticancer drug in combination.

### Solution to Problem

As a result of studies to solve the above-described problems, the present inventors have revealed that the combination of an antibody which specifically recognizes the mutated amino acid sequence of CALR and a specific anticancer drug can dramatically improve the antitumor effect and provide an excellent therapeutic effect, and have found that a pharmaceutical comprising an antibody which specifically binds to a mutant CALR protein and a specific anticancer drug in combination is useful as a more effective pharmaceutical for preventing and/or treating a cancer, thereby completing the present invention.

Accordingly, the present invention provides the following (1) to (10).
(1) A pharmaceutical for preventing and/or treating a cancer, comprising (A) an antibody which specifically binds to a mutant calreticulin protein or a functional fragment thereof and (B) at least one drug selected from the group consisting of an alkylating agent, a platinum preparation, an antimetabolite, a ribonucleotide reductase inhibitor, a nucleotide analog, a topoisomerase inhibitor, a microtubule polymerization inhibitor, a Bcl-2 inhibitor, an antitumor antibiotic, an interferon, a cytokine preparation, a molecular targeting drug, a nucleic acid synthesis inhibitor, a JAK inhibitor, and a cancer immunotherapeutic agent in combination.
(2) The pharmaceutical according to (1), wherein the component (A) is an antibody or a functional fragment thereof which does not bind to a wild-type calreticulin protein but has high affinity to the mutant calreticulin protein.
(3) The pharmaceutical according to (1) or (2), wherein the component (A) is an antibody or a functional fragment thereof which binds to a mutant amino acid sequence of SEQ ID NO: 2 or 3 or a sequence having 80% or more homology to the mutant amino acid sequence.
(4) The pharmaceutical according to any one of (1) to (3), wherein the component (A) is at least one antibody or a functional fragment thereof selected from the group consisting of a human antibody, a humanized antibody, a bispecific antibody, a multispecific antibody, a chimeric antibody of an animal-derived antibody and a human antibody, Fab, Fab', F(ab')₂, a single-chain antibody (scFv), and a diabody.
(5) The pharmaceutical according to any one of (1) to (4), wherein the component (B) is at least one drug selected from the group consisting of a JAK inhibitor, a nucleic acid synthesis inhibitor, an interferon, a cytokine preparation, a molecular targeting drug, and a cancer immunotherapeutic agent.
(6) The pharmaceutical according to any one of (1) to (5), wherein the component (B) is at least one drug selected from the group consisting of ruxolitinib, hydroxyurea, interferon α, and a cancer immunocheckpoint inhibitor.
(7) The pharmaceutical according to any one of (1) to (6), wherein the cancer is selected from the group consisting of lymphoma, leukemia, multiple myeloma, a myeloproliferative neoplasm (MPN), gastrointestinal cancer such as esophageal cancer, gastric cancer, and duodenal cancer, liver cancer, biliary tract cancer such as gallbladder/bile duct cancer, pancreatic cancer, small intestinal cancer, large intestine cancer such as colorectal cancer, colon cancer, and rectal cancer, gastrointestinal stromal tumor, lung cancer such as non-small cell lung cancer and small-cell lung cancer, and kidney cancer.
(8) Use of a combination of (A) an antibody which specifically binds to a mutant calreticulin protein or a functional fragment thereof and (B) at least one drug selected from the group consisting of an alkylating agent, a platinum preparation, an antimetabolite, a ribonucleotide reductase inhibitor, a nucleotide analog, a topoisomerase inhibitor, a microtubule polymerization inhibitor, a Bcl-2 inhibitor, an antitumor antibiotic, an interferon, a cytokine preparation, a molecular targeting drug, a nucleic acid synthesis inhibitor, a JAK inhibitor, and a cancer immunotherapeutic agent, for producing a pharmaceutical for preventing and/or treating a cancer.
(9) A combination of (A) an antibody which specifically binds to a mutant calreticulin protein or a functional fragment thereof and (B) at least one drug selected from the group consisting of an alkylating agent, a platinum preparation, an antimetabolite, a ribonucleotide reductase inhibitor, a nucleotide analog, a topoisomerase inhibitor, a microtubule polymerization inhibitor, a Bcl-2 inhibitor, an antitumor antibiotic, an interferon, a cytokine preparation, a molecular targeting drug, a nucleic acid synthesis inhibitor, a JAK inhibitor, and a cancer immunotherapeutic agent, for use in preventing and/or treating a cancer.
(10) A method for preventing and/or treating a cancer, comprising administering a combination of (A) an antibody which specifically binds to a mutant calreticulin protein or a functional fragment thereof and (B) at least one drug selected from the group consisting of an alkylating agent, a platinum preparation, an antimetabolite, a ribonucleotide reductase inhibitor, a nucleotide analog, a topoisomerase inhibitor, a microtubule polymerization inhibitor, a Bcl-2 inhibitor, an antitumor antibiotic, an interferon, a cytokine preparation, a molecular targeting drug, a nucleic acid synthesis inhibitor, a JAK inhibitor, and a cancer immunotherapeutic agent in combination.

### Advantageous Effects of Invention

The antibody used in the present invention has an antigenic recognition site (epitope) in the mutant CALR polypeptide chain. When used in combination with the anticancer drug, the antibody effectively injures cancer cells compared to the case where each of them is administered alone, and provides a dramatically improved antitumor effect. Therefore, the pharmaceutical of the present invention can be used to specifically prevent and/or treat various cancers, especially diseases with expression of a mutant CALR.

### Brief Description of Drawings

[FIG.1] FIG.1 is a diagram showing the characteristics of mutant CALR proteins. The CALR gene mutation responsible for the development of MPN in MPN patients is +1 frameshift mutation, and an amino acid sequence common to mutant proteins is found in the C-terminus of the mutant CALR proteins produced as the results of the mutation. SP: signal sequence, N: N domain, and P: P domain. The arrow indicates where the amino acid sequences different from the wild-type (WT) sequence start.

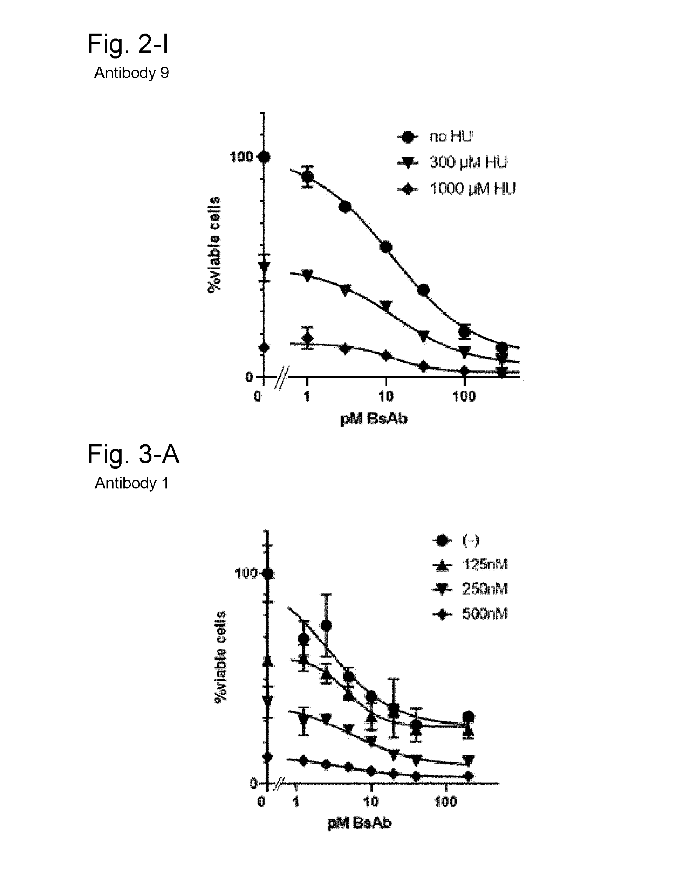

[FIG.2-A] FIG.2-A shows the combination effect of an anti-mutant CALR/CD3 bispecific antibody (Antibody No. 1) and hydroxyurea. UT-7/TPO/Ins 5-Luc cells (target cells) expressing mutant CALR and luciferase were cultured with human peripheral T cells in the presence of a CALR/CD3 bispecific antibody and hydroxyurea, and then the viability of the target cells was measured using luciferase activity as an indicator to evaluate T cell-dependent cellular cytotoxicity (hereinafter referred to as TDCC). The cell viability of each well was calculated using the chemiluminescence value of the group without a CALR/CD3 bispecific antibody and a combined drug as 100%.
[FIG.2-B] FIG.2-B shows the combination effect of an anti-mutant CALR/CD3 bispecific antibody (Antibody No. 2) and hydroxyurea.
[FIG.2-C] FIG.2-C shows the combination effect of an anti-mutant CALR/CD3 bispecific antibody (Antibody No. 3) and hydroxyurea.
[FIG.2-D] FIG.2-D shows the combination effect of an anti-mutant CALR/CD3 bispecific antibody (Antibody No. 4) and hydroxyurea.
[FIG.2-E] FIG.2-E shows the combination effect of an anti-mutant CALR/CD3 bispecific antibody (Antibody No. 5) and hydroxyurea.
[FIG.2-F] FIG.2-F shows the combination effect of an anti-mutant CALR/CD3 bispecific antibody (Antibody No. 6) and hydroxyurea.
[FIG.2-G] FIG.2-G shows the combination effect of an anti-mutant CALR/CD3 bispecific antibody (Antibody No. 7) and hydroxyurea.
[FIG.2-H] FIG.2-H shows the combination effect of an anti-mutant CALR/CD3 bispecific antibody (Antibody No. 8) and hydroxyurea.

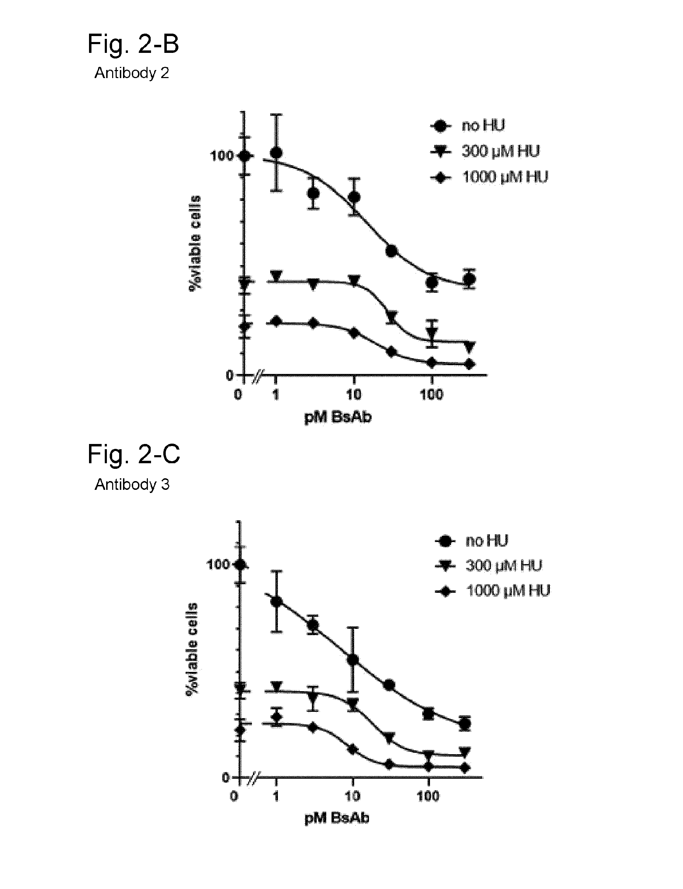

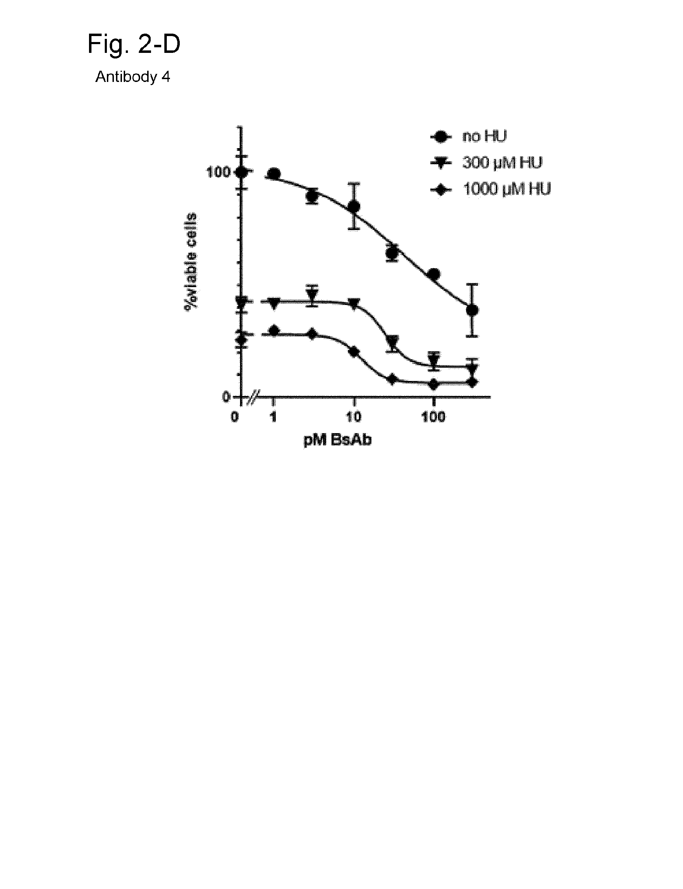

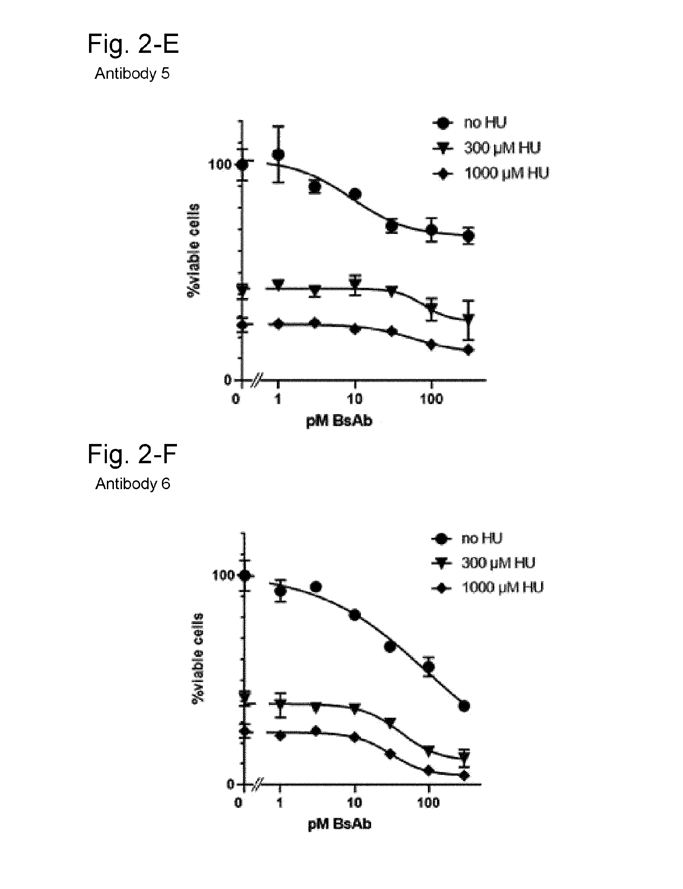

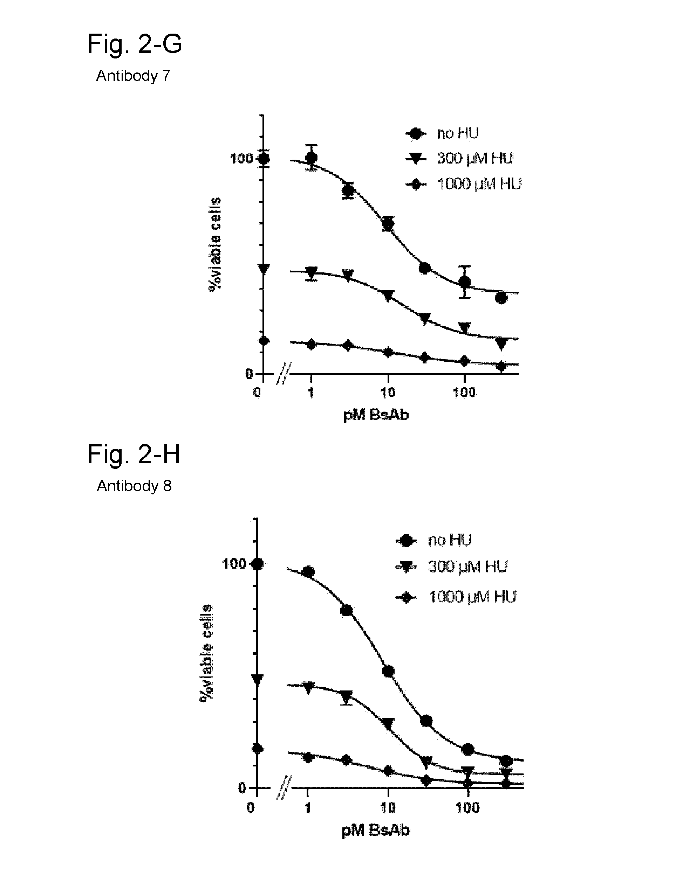

[FIG.2-I] FIG.2-I shows the combination effect of an anti-mutant CALR/CD3 bispecific antibody (Antibody No. 9) and hydroxyurea.
[FIG.3-A] FIG.3-A shows the combination effect of an anti-mutant CALR/CD3 bispecific antibody (Antibody No. 1) and ruxolitinib on in vitro efficacy. UT-7/TPO/Ins 5-GFP-Luc cells (target cells) expressing mutant CALR and luciferase were cultured with human peripheral T cells in the presence of a CALR/CD3 bispecific antibody and ruxolitinib, and then the viability of the target cells was measured using luciferase activity as an indicator to evaluate T cell-dependent cellular cytotoxicity (hereinafter referred to as TDCC). The cell viability of each well was calculated using the chemiluminescence value of the group without a CALR/CD3 bispecific antibody (Antibody No. 1) and a combined drug as 100%.

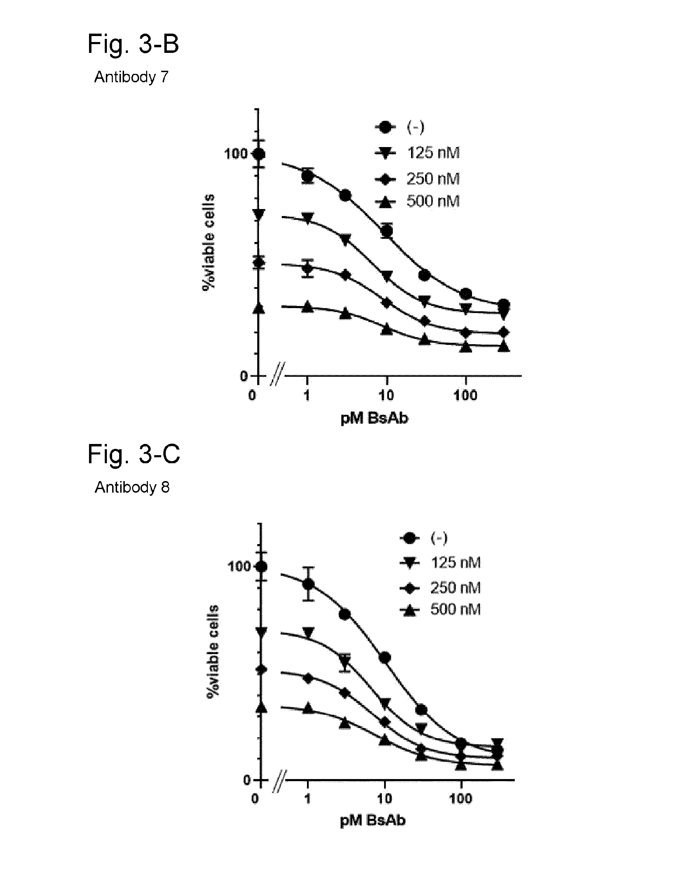

[FIG.3-B] FIG.3-B shows the combination effect of an anti-mutant CALR/CD3 bispecific antibody (Antibody No. 7) and ruxolitinib.
[FIG.3-C] FIG.3-C shows the combination effect of an anti-mutant CALR/CD3 bispecific antibody (Antibody No. 8) and ruxolitinib.

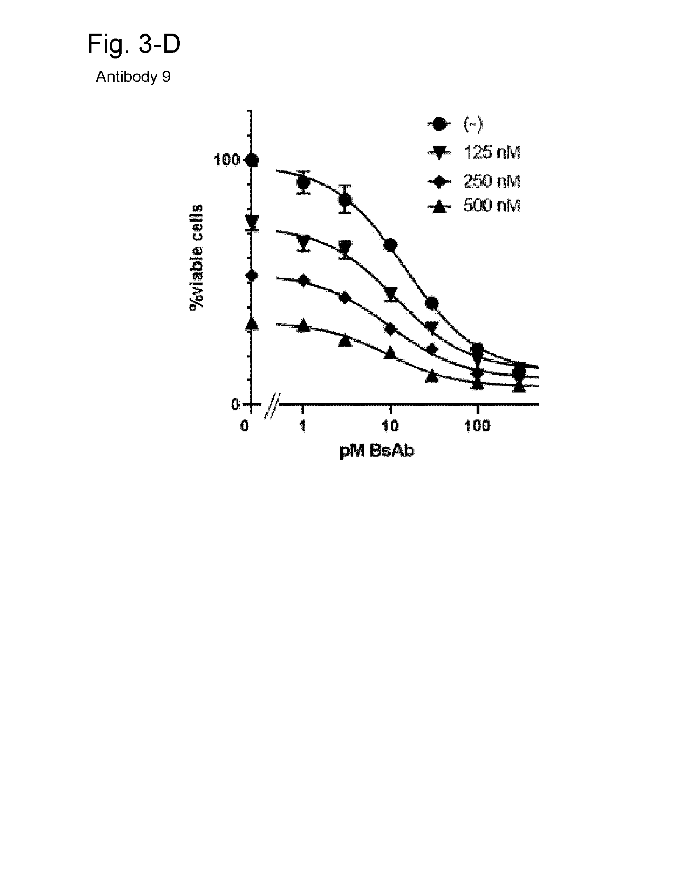

[FIG.3-D] FIG.3-D shows the combination effect of an anti-mutant CALR/CD3 bispecific antibody (Antibody No. 9) and ruxolitinib.

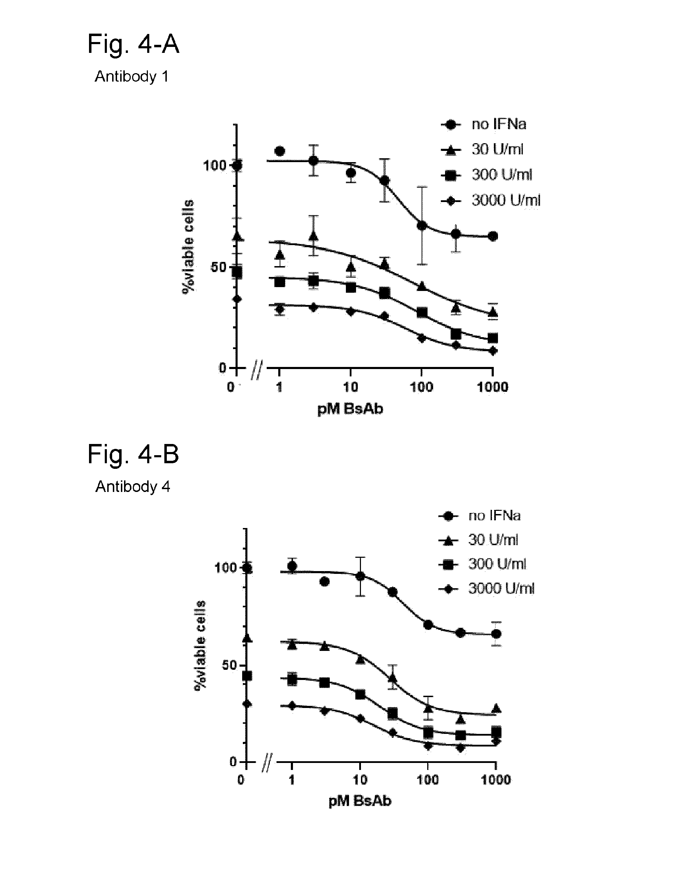

[FIG.4-A] FIG.4-A shows the combination effect of an anti-mutant CALR/CD3 bispecific antibody (Antibody No. 1) and interferon α. UT-7/TPO/Ins 5-Luc cells (target cells) expressing mutant CALR and luciferase were cultured with human peripheral T cells in the presence of a CALR/CD3 bispecific antibody and interferon-α (IFN-α), and then the viability of the target cells was measured using luciferase activity as an indicator to evaluate T cell-dependent cellular cytotoxicity (hereinafter referred to as TDCC). The cell viability of each well was calculated using the chemiluminescence value of the group without a CALR/CD3 bispecific antibody and a combined drug as 100%.
[FIG.4-B] FIG.4-B shows the combination effect of an anti-mutant CALR/CD3 bispecific antibody (Antibody No. 4) and IFN-α.

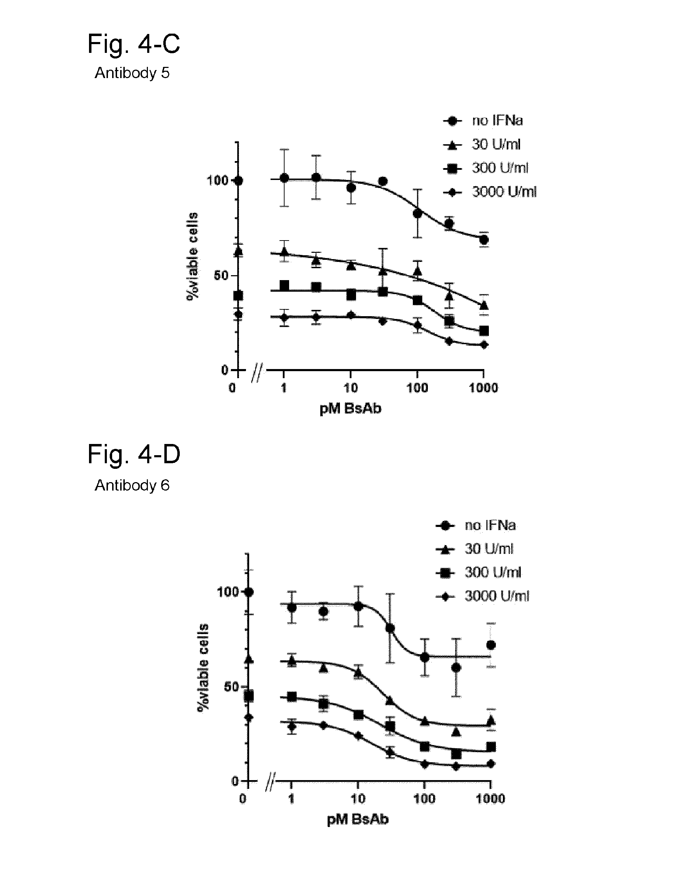

[FIG.4-C] FIG.4-C shows the combination effect of an anti-mutant CALR/CD3 bispecific antibody (Antibody No. 5) and IFN-α.
[FIG.4-D] FIG.4-D shows the combination effect of an anti-mutant CALR/CD3 bispecific antibody (Antibody No. 6) and IFN-α.

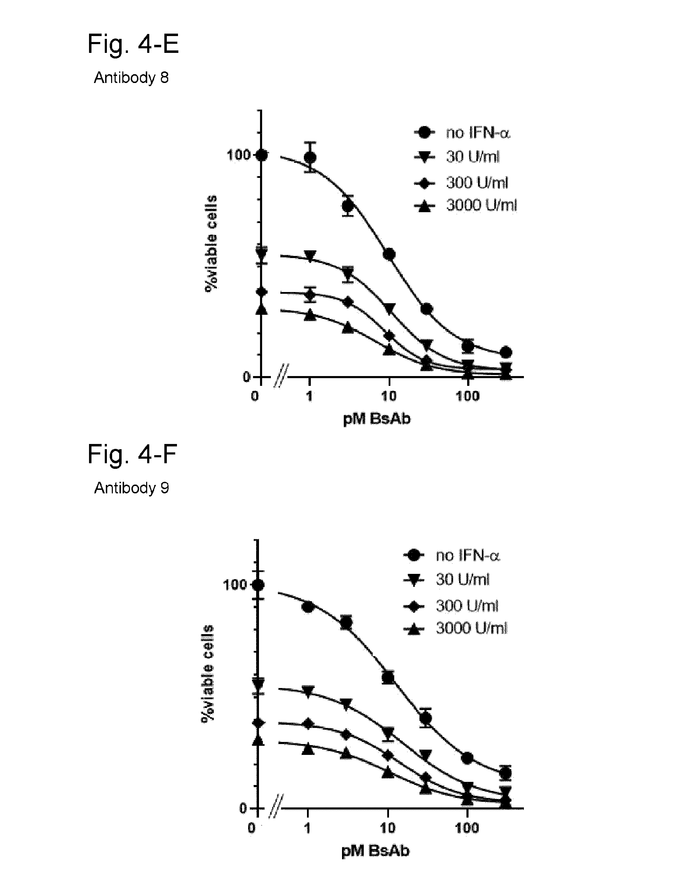

[FIG.4-E] FIG.4-E shows the combination effect of an anti-mutant CALR/CD3 bispecific antibody (Antibody No. 8) and IFN-α.
[FIG.4-F] FIG.4-F shows the combination effect of an anti-mutant CALR/CD3 bispecific antibody (Antibody No. 9) and IFN-α.

### Description of Embodiments

The present invention is based on a finding that cancer can be effectively treated and/or prevented by combining, with an anticancer agent, an antibody which recognizes 13 amino acids (SEQ ID NO: 4) in the C-terminal polypeptide (Figure 1) which remains after cleavage in a sequence specific to a mutant CALR protein, used in methods for detecting and diagnosing mutant CALR proteins related to an MPN and for preventing or treating an MPN, or an antibody which recognizes a sequence (SEQ ID NO: 5) located closer to the C-terminal side than the sequence.

In other words, the present invention provides a pharmaceutical for preventing and/or treating a cancer, especially a pharmaceutical for preventing and/or treating MPN, comprising (A) an antibody which specifically recognizes a mutant CALR protein and (B) an anticancer agent in combination.

Component (A) used in the pharmaceutical of the present invention is an antibody which specifically recognizes the mutant CALR protein.

As used herein, the antibody or functional fragment thereof means a protein which specifically binds to mutant CALR, including monoclonal antibodies and chimeric antibodies, humanized antibodies, human antibodies, bispecific antibodies, multispecific antibodies, engager, Fab, Fab', F(ab')₂, single-chain antibodies (scFv), diabodies, minibodies, a protein fragment, and a derivative containing the protein fragment thereof produced by genetic recombination techniques.

The forms of the monoclonal antibody include human and non-human animal IgG, IgM, IgA, IgE, and IgD.

The forms of the bispecific and multispecific antibodies are not particularly limited and may be any form known in the art, or may be any form as long as it retains specificity for the two or more antigens.

The form of the bispecific antibody is broadly classified into IgG-like type and low molecular weight type. An IgG-like type is a form that retains the Fc region. Examples of the forms of IgG-like antibodies include, but are not limited to, CrossMab, DAF(two-in-one), DAF(four-in-one), DutaMab, DT-IgG, knobs-into-holes, knobs-into-holes common LC, SEEDbody, Triomab, κλ-body, DVD-Ig, IgG-scFv, and DuoBody. Since the IgG-like antibodies retain the Fc region, they are expected to, for example, exhibit effector functions such as ADCC and CDC, facilitate purification, improve stability, and extend the half-life in the blood. On the other hand, the low molecular weight type usually has an Fv region composed of a heavy chain variable region and a light chain variable region as its basic component. Examples of the forms of the low molecular weight antibodies include, but are not limited to, Diabody(Db), BiTE, DART, TandAb, scDb, triple body, mini antibody, minibody, scFv, tandem scFv, and F(ab')₂. The low molecular weight type is expected to have improved tissue permeability and high productivity due to its size. In addition, modified antibodies are also included, such as those in which the amino acid sequence has a deletion, substitution or addition while retaining the ability to bind to an antigen, those in which part or all of the sugar chain is deleted or added, those in which a linker or the like is added, those fused with other proteins, and antibody-drug conjugates (ADC) in which the antibody is bound to a low molecular weight pharmaceutical via a linker.

The antibody of the present invention has a domain which specifically binds to a mutant calreticulin protein.

The domain competes with an antibody which binds to an epitope of a mutant calreticulin protein of SEQ ID NO: 4 or 5. Here, the mutant calreticulin protein preferably has an antigenic recognition site in a polypeptide chain consisting of an amino acid sequence of SEQ ID NO: 4 or 5 or a polypeptide chain consisting of an amino acid sequence of SEQ ID NO: 4 or 5 with deletion, substitution, or addition of one or several amino acids.

The domain does not bind to a wild-type calreticulin protein but has high affinity to the mutant calreticulin protein.

Examples of cleaved CALR proteins include (i) a polypeptide consisting of an amino acid sequence of SEQ ID NO: 4 or 5 and (ii) a polypeptide consisting of an amino acid sequence of SEQ ID NO: 4 or 5 with deletion, substitution, or addition of one or several amino acids. In (ii), an amino acid sequence in which 1 to 4 amino acids are deleted, substituted, or added is preferred, and an amino acid sequence in which 1 to 3 amino acids are deleted, substituted, or added is more preferred. The identity of the amino acid sequence of the polypeptide (ii) and the amino acid sequence of SEQ ID NO: 1 is preferably 80% or more, more preferably 85% or more, and even more preferably 90% or more.

The binding domain of the antibody may be any antibody which recognizes an antigen-recognition site (epitope) in the polypeptide chain (i) or (ii), and may bind only to a cleaved mutant CALR protein or may bind to both the cleaved mutant CALR protein and a full-length mutant CALR protein. The antibody is preferably an antibody specific to the "mutant CALR protein" which binds to both the cleaved mutant CALR protein and the full-length mutant CALR protein.

Such binding domains are preferably heavy-chain and light-chain variable regions of anti-mutant CALR antibodies (clone B3, C6, and G1 antibodies) that the present inventors found, as well as heavy-chain complementarity determining regions (CDR-H1, CDR-H2, and CDR-H3) and light-chain complementarity determining regions (CDR-L1, CDR-L2, and CDR-L3).

The first domain in the bispecific antibody is preferably a heavy-chain variable region and light-chain variable region of the anti-mutant CALR antibodies (clone B3, C6, and G1 antibodies), as well as a heavy-chain complementarity determining region (CDR-H1, CDR-H2, and CDR-H3) and a light-chain complementarity determining region (CDR-L1, CDR-L2, and CDR-L3).

The second domain in the bispecific antibody is preferably a domain which specifically binds to a CD3 antigen and is preferably a heavy-chain variable region and light-chain variable region derived from existing anti-CD3 antibodies, as well as a heavy-chain complementarity determining region (CDR-H1, CDR-H2, and CDR-H3) and a light-chain complementarity determining region (CDR-L1, CDR-L2, and CDR-L3), derived from an existing anti-CD3 antibody. Examples of the existing anti-CD3 antibodies include OKT3 and UCTH1.

Preferred examples of the antibodies of the present invention include heavy-chain and light-chain variable regions of anti-mutant CALR antibodies (clone B3, C6, and G1 antibodies), as well as heavy-chain complementarity determining regions (CDR-H1, CDR-H2, and CDR-H3) and light-chain complementarity determining regions (CDR-L1, CDR-L2, and CDR-L3), and Table 1 shows examples of amino acid sequences thereof.

As preferred examples of the first and second domains of the bispecific antibodies, Tables 1 shows amino acid sequences of heavy-chain and light-chain variable regions of anti-mutant CALR antibodies (clone B3, C6, and G1 antibodies) and anti-CD3 antibodies (OKT3 and UCHT1), as well as amino acid sequences of heavy-chain complementarity determining regions (CDR-H1, CDR-H2, and CDR-H3) and light-chain complementarity determining regions (CDR-L1, CDR-L2, and CDR-L3).

**[Table 1]**

| SEQ ID NO | Name | Amino acid sequence |
|---|---|---|
| 6 | B3 VH | QVQLQQSGAELVKPGSSVKISCKASGYTFTRNF IHWIKQQPGNGLEWIGWIFPGDGDTEYNQKFN GKATLTADKSSSTAYMQLSSLTSEDSAVYFCAR GNYNYEYFDYWGQGVMVTVSS |
| 7 | B3 VL | DIQMTQSPASLSASLGETVSIECLASEDIYSYLA WYQQKPGKSPQLLIFAANRLQDGVPSRFSGSG SGTQFSLKISGMQPEDEGDYFCLQGSKFPYTF GPGTKLELN |
| 8 | C6 VH | QVQLQQSGAELVKPGSSVKISCKASGYTFTSYF VHWIKQQPGDGLEWIGWIYPGDGDTEYNHKFN GKSTLTADRSSSTAYMQLSSLTSEDSAVYFCAR GNYYDGREVMDAWGQGASVTVSS |
| 9 | C6 VL | DVVLTQTPPTLSATIGQSVSISCRSSQSLLDSDG ETYLNWLLQRPGQSPQLLIYSVSNLESGVPNRF SGSGSETDFTLKISGMEAEDLGVYYCMQATHG PYTFGAGTKLELK |
| 10 | G1 VH | QVQLQQSGAELVKPGSSVKISCKASGYTFTSYF VHWIKQQPGDGLEWIGWIYPGDGDTEYNQKFN GKATLTADRSSSTAYMQLSSLTSEDSAVYFCAR GNYYDGREVMDAWGQGASVTVSS |
| 11 | G1 VL | DVVLTQTPPTLSATIGQSVSISCRSSQSLLDSDG ETYLNWLLQRPGQCPQLLIYSVSNLESGVPNRF SGSGSETDFTLKISGVEAEDLGVYYCMQGTHG PYTFGAGTKLELK |
| 12 | OKT3 VH | QVQLVQSGGGVVQPGRSLRLSCKASGYTFTRY TMHWVRQAPGKGLEWIGYINPSRGYTNYNQKV KDRFTISRDNSKNTAFLQMDSLRPEDTGVYFCA RYYDDHYSLDYWGQGTPVTVSS |
| 13 | OKT3 VL | DIQMTQSPSSLSASVGDRVTITCSASSSVSYMN WYQQTPGKAPKRWIYDTSKLASGVPSRFSGSG SGTDYTFTISSLQPEDIATYYCQQWSSNPFTFG QGTKLQITR |
| 14 | B3 CDR-H1 | GYTFTRNFIH |
| 15 | B3 CDR-H2 | WIFPGDGDTEYNQKFNG |
| 16 | B3 CDR-H3 | GNYNYEYFDY |
| 17 | B3 CDR-L1 | LASEDIYSYLA |
| 18 | B3 CDR-L2 | AANRLQD |
| 19 | B3 CDR-L3 | LQGSKFPYT |
| 20 | C6 CDR-H1 | GYTFTSYFVH |
| 21 | C6 CDR-H2 | WIYPGDGDTEYNHKFNG |
| 22 | C6 CDR-H3 | GNYYDGREVMDA |
| 23 | C6 CDR-L1 | RSSQSLLDSDGETYLN |
| 24 | C6 CDR-L2 | SVSNLES |
| 25 | C6 CDR-L3 | MQATHGPYT |
| 26 | G1 CDR-H1 | GYTFTSYFVH |
| 27 | G1 CDR-H2 | WIYPGDGDTEYNQKFNG |
| 28 | G1 CDR-H3 | GNYYDGREVMDA |
| 29 | G1 CDR-L1 | RSSQSLLDSDGETYLN |
| 30 | G1 CDR-L2 | SVSNLES |
| 31 | G1 CDR-L3 | MQGTHGPYT |
| 32 | OKT3 CDR-H1 | RYTMH |
| 33 | OKT3 CDR-H2 | YINPSRGYTNYNQKVKD |
| 34 | OKT3 CDR-H3 | YYDDHYSLDY |
| 35 | OKT3 CDR-L1 | SASSSVSYMN |
| 36 | OKT3 CDR-L2 | DTSKLAS |
| 37 | OKT3 CDR-L3 | QQWSSNPFT |
| 217 | UCHT1 VH | |
| 218 | UCHT1 VL | |
| 219 | UCHT1 CDR-H1 | GYTMN |
| 220 | UCHT1 CDR-H2 | LINPYKGVSTYNQKFKD |
| 221 | UCHT1 CDR-H3 | SGYYGDSDWYFDV |
| 222 | UCHT1 CDR-L1 | RASQDIRNYLN |
| 223 | UCHT1 CDR-L2 | YTSRLES |
| 224 | UCHT1 CDR-L3 | QQGNTLPWT |

These regions can be made as recombinant antibodies by genetic engineering techniques based on sequence information of antibodies obtained by immunizing animals with a part or the whole length of the cleaved mutant CALR protein or by sensitizing lymphocytes in vitro and then fusing it with myeloma, and antibodies obtained, for example, from antibody libraries such as phage display. The sequence information related to anti-CD3 antibodies can be obtained from existing information. In other words, heavy-chain and light-chain genes of the antibodies are synthesized from the obtained genetic information, inserted into a vector (e.g., plasmid), and then transfected into host cells (e.g., CHO cells, HEK cells, and the like.) to culture the host cells, thereby collecting the recombinant antibodies from the culture. In the synthesis of heavy-chain and light-chain genes of the antibodies, it is preferable to optimize codons.

Examples of preferred bispecific antibodies of the present invention include bispecific antibodies in which the CDR of the first domain has (a) an amino acid sequence having 80% or more homology to the amino acid sequences of SEQ ID NO: 14 to 16 (VHCDR), and an amino acid sequence having 80% or more homology to the amino acid sequences of SEQ ID NO: 17 to 19 (VLCDR); (b) an amino acid sequence having 80% or more homology to the amino acid sequences of SEQ ID NO: 20 to 22 (VHCDR), and an amino acid sequence having 80% or more homology to the amino acid sequences of SEQ ID NO: 23 to 25 (VLCDR); or (c) an amino acid sequence having 80% or more homology to the amino acid sequences of SEQ ID NO: 26 to 28 (VHCDR), and an amino acid sequence having 80% or more homology to the amino acid sequences of SEQ ID NO: 29 to 31 (VLCDR).

Furthermore, examples thereof include bispecific antibodies in which the first domain has (d) a VH region having an amino acid sequence having 80% or more homology to the amino acid sequences of SEQ ID NO: 6, and a VL region having an amino acid sequence having 80% or more homology to the amino acid sequences of SEQ ID NO: 7; (e) a VH region having an amino acid sequence having 80% or more homology to the amino acid sequence of SEQ ID NO: 8, and a VL region having an amino acid sequence having 80% or more homology to the amino acid sequence of SEQ ID NO: 9; or (f) a VH region having an amino acid sequence having 80% or more homology to the amino acid sequence of SEQ ID NO: 10, and a VL region having an amino acid sequence having 80% or more homology to the amino acid sequence of SEQ ID NO: 11.

A bispecific antibody is preferred in which the CDR of the second domain has an amino acid sequence having 80% or more homology to the amino acid sequences of SEQ ID NO: 32 to 34 (VHCDR) and an amino acid sequence having 80% or more homology to the amino acid sequences of SEQ ID NO: 35-37 (VLCDR); or an amino acid sequence having 80% or more homology to the amino acid sequences of SEQ ID NO: 219 to 221 (VHCDR) and an amino acid sequence having 80% or more homology to the amino acid sequences of SEQ ID NO: 222 to 224 (VLCDR).

Furthermore, a bispecific antibody is preferred in which the second domain has a VH region having an amino acid sequence having 80% or more homology to the amino acid sequence of SEQ ID NO: 12, and a VL region having an amino acid sequence having 80% or more homology to the amino acid sequence of SEQ ID NO: 13; or a VH region having an amino acid sequence having 80% or more homology to the amino acid sequence of SEQ ID NO: 217, and a VL region having an amino acid sequence having 80% or more homology to the amino acid sequence of SEQ ID NO: 218.

Examples of more preferred bispecific antibodies of the present invention include bispecific antibodies in which the CDR of the first domain has (a) an amino acid sequence having 80% or more homology to the amino acid sequences of SEQ ID NO: 14 to 16 (VHCDR), and an amino acid sequence having 80% or more homology to the amino acid sequences of SEQ ID NO: 17 to 19 (VLCDR); (b) an amino acid sequence having 80% or more homology to the amino acid sequences of SEQ ID NO: 20 to 22 (VHCDR), and an amino acid sequence having 80% or more homology to the amino acid sequences of SEQ ID NO: 23 to 25 (VLCDR); or (c) an amino acid sequence having 80% or more homology to the amino acid sequences of SEQ ID NO: 26 to 28 (VHCDR), and an amino acid sequence having 80% or more homology to the amino acid sequences of SEQ ID NO: 29 to 31 (VLCDR), and the CDR of the second domain has an amino acid sequence having 80% or more homology to the amino acid sequences of SEQ ID NO: 32 to 34 (VHCDR), and an amino acid sequence having 80% or more homology to the amino acid sequences of SEQ ID NO: 35 to 37 (VLCDR); or an amino acid sequence having 80% or more homology to the amino acid sequences of SEQ ID NO: 219 to 221 (VHCDR), and an amino acid sequence having 80% or more homology to the amino acid sequences of SEQ ID NO: 222-224 (VLCDR).

Examples of further preferred bispecific antibodies include bispecific antibodies in which the first domain has (d) a VH region having an amino acid sequence having 80% or more homology to the amino acid sequence of SEQ ID NO: 6, and a VL region having an amino acid sequence having 80% or more homology to the amino acid sequence of SEQ ID NO: 7; (e) a VH region having an amino acid sequence having 80% or more homology to the amino acid sequence of SEQ ID NO: 8, and a VL region having an amino acid sequence having 80% or more homology to the amino acid sequence of SEQ ID NO: 9; or (f) a VH region having an amino acid sequence having 80% or more homology to the amino acid sequence of SEQ ID NO: 10, and a VL region having an amino acid sequence having 80% or more homology to the amino acid sequence of SEQ ID NO: 11, and the second domain has (g) a VH region having an amino acid sequence having 80% or more homology to the amino acid sequence of SEQ ID NO: 12, and a VL region having an amino acid sequence having 80% or more homology to the amino acid sequence of SEQ ID NO: 13; or (h) a VH region having an amino acid sequence having 80% or more homology to the amino acid sequence of SEQ ID NO: 217, and a VL region having an amino acid sequence having 80% or more homology to the amino acid sequence of SEQ ID NO: 218.

As described above, the form of the antibody of the present invention may be any form as long as it retains specificity for the two antigens, and may be an IgG-like type or a low molecular weight type. Specifically, it may have an Fc region, a hetero-H chain, or the like.

Specific examples of the bispecific antibody include antibodies selected from the group consisting of the following (I) to (IX).
(I) A bispecific antibody comprising an H chain containing an amino acid sequence of SEQ ID NO: 174 and an L chain containing an amino acid sequence of SEQ ID NO: 202 (Antibody 1).
(II) A bispecific antibody comprising an H chain containing an amino acid sequence of SEQ ID NO: 186 and an L chain containing an amino acid sequence of SEQ ID NO: 201 (Antibody 2).
(III) A bispecific antibody comprising an H chain containing an amino acid sequence of SEQ ID NO: 174 and an L chain containing an amino acid sequence of SEQ ID NO: 201 (Antibody 3).
(IV) A bispecific antibody comprising an H chain containing an amino acid sequence of SEQ ID NO: 180 and an L chain containing an amino acid sequence of SEQ ID NO: 202 (Antibody 4).
(V) A bispecific antibody comprising an H chain containing an amino acid sequence of SEQ ID NO: 181 and an L chain containing an amino acid sequence of SEQ ID NO: 202 (Antibody 5).
(VI) A bispecific antibody comprising an H chain containing an amino acid sequence of SEQ ID NO: 161 and an L chain containing an amino acid sequence of SEQ ID NO: 171 (Antibody 6).
(VII) A bispecific antibody comprising an H chain containing an amino acid sequence of SEQ ID NO: 225 and an L chain containing an amino acid sequence of SEQ ID NO: 202 (Antibody 7).
   The antibodies (I) to (VII) above all have the same structure and are bispecific antibodies wherein the Fc is further fused to a polypeptide in which the single-chain Fv that specifically binds to CD3 is fused to a heavy chain C-terminus of the Fab fragment that specifically binds to the mutant CALR via a peptide linker.
(VIII) A bispecific antibody consisting of the amino acid sequence of SEQ ID NO: 226 (Antibody 8).
(IX) A bispecific antibody comprising an H chain containing the amino acid sequence of SEQ ID NO: 227 and an L chain containing the amino acid sequence of SEQ ID NO: 228, wherein the light chain N-terminus of the IgG that specifically binds to the mutant CALR is fused to the C-terminus of the single-chain Fv that specifically binds to the CD3 antigen via a peptide linker (Antibody 9) .

Preferred specific examples of the bispecific antibodies of the present invention include those having each of the sequences, and amino acid sequences in the regions other than these regions are not particularly restricted. The antibody of the present invention may be a humanized antibody and a non-human mammalian antibody. More specifically, the antibody may be a chimeric antibody composed of the variable regions of the heavy chain and light chain of an antibody of a non-human mammal, for example, a rat, and the constant regions of the heavy chain and light chain of a human antibody, and such an antibody can be obtained by ligating a DNA encoding the variable region of a rat antibody with a DNA encoding the constant region of a human antibody, incorporating this into an expression vector and introducing the vector into a host for production. As an example of the humanized antibody, VH 1 to 5 and VL 1 to 5 are shown below (Table 2).

**[Table 2]**

| SEQ ID NO | Name | Amino acid sequence |
|---|---|---|
| 94 | VH1 | QVQLVQSGAEVKKPGASVKVSCKASGYTFTRNFIHWVRQA PGQGLEWMGWIFPGDGDTEYNQKFNGRVTMTRDTSISTAY MELSRLRSDDTAVYYCARGNYNYEYFDYWGQGTMVTVSS |
| 95 | VH2 | QVQLQESGAEVKKPGASVKVSCKASGYTFTRNFIHWVRQA PGQRLEWMGWIFPGDGDTEYNQKFNGRVTITRDTSASTAY MELSSLRSEDTAVYYCARGNYNYEYFDYWGQGTLVTVSS |
| 96 | VH3 | QVQLVQSGAEVKKPGASVKISCKASGYTFTRNFIHWVRQA PGQSLEWVGWIFPGDGDTEYNQKFNGRITITRDTSANTAY MELSSLKSEDTAVYYCARGNYNYEYFDYWGQGTLVTVSS |
| 97 | VH4 | QMQLVQSGAEVKKTGSSVKVSCKASGYTFTRNFIHWVRQA PGQALEWMGWIFPGDGDTEYNQKFNGRVTITRDRSMSTAY MELSSLRSEDTAMYYCARGNYNYEYFDYWGQGTLVTVSS |
| 98 | VH5 | QVQLVQSGAEVKKPGSSVKVSCKASGYTFTRNFIHWVRQQ PGQGLEWMGWIFPGDGDTEYNQKFNGRVTITADKSTSTAY MQLSSLTSEDTAVYFCARGNYNYEYFDYWGQGVMVTVSS |
| 99 | VL1 | DIQMTQSPSFVSASVGDRVTITCLASEDIYSYLAWYQQKPG KAPKLLIFAANRLQDGVPSRFSGGGSGTGFTLTISSLQPEDF ATYFCLQGSKFPYTFGQGTKLEIN |
| 100 | VL2 | DIQLTQSPAFLSASLGDRVTITCLASEDIYSYLAWYQQKPGK APKLLISAANRLQDGVPSRFSGSGSGTEFTLTISSLQPEDFA TYSCLQGSKFPYTFGQGTKLEIK |
| 101 | VL3 | DIQLTQSPSSLSASVGDRVTITCLASEDIYSYLAWYQQKPGK GPKLLIYAANRLQDGVPSRFSGSGSGTDFSLTISSLQPEDLA TYYCLQGSKFPYTFGGGTKVEIK |
| 102 | VL4 | DIQMTQSPSSLSASVGDTVTIACLASEDIYSYLAWYQQKPG KAPKLLIYAANRLQDGVPSRFSGSGSFTDFTLTINSLQPDDS ATYYCLQGSKFPYTFGGGTKVDIK |
| 103 | VL5 | DIQMTQSPSSVSASVGDSVTIECLASEDIYSYLAWYQQKPG KSPKLLIFAANRLQDGVPSRFSGSGSGTQFTLKISSLQPEDF AEYFCLQASKFPYTFGPGTKLELN |

Another method for obtaining human antibodies is also known. For example, human lymphocytes are sensitized with a desired antigen or a cell expressing a desired antigen in vitro, and the sensitized lymphocytes are fused with human myeloma cells, such as U266, to obtain a desired human antibody having a binding activity to the antigen (See JP-B-1-59878). A desired human antibody can be obtained by immunizing a transgenic animal having all repertories of human antibody genes with a desired antigen (See WO93/12227, WO92/03918, WO94/02602, WO94/25585, WO96/34096, and WO96/33735). Furthermore, a technique of obtaining a human antibody by panning using a human antibody library is also known. For example, a variable region of a human antibody is expressed as a single-chain antibody (scFv) on the surface of the phage by a phage display method, and the phage binding to the antigen can be selected. By analyzing the gene of the selected phage, the DNA sequence encoding the variable region of the human antibody which binds to the antigen can be determined. If the DNA sequence of the scFv which binds to the antigen is revealed, a human antibody can be obtained by making an appropriate expression vector with the sequence. These methods are already well known, and WO92/01047, WO92/20791, WO93/06213, WO93/11236, WO93/19172, WO95/01438, and WO95/15388 can be referred to.

For the bispecific antibodies, bispecific antibody expression vectors can be constructed using, for example, DNA encoding anti-mutant CALR and anti-CD3 antibodies and expression vectors, and the expression vectors can be transfected into CHO cells or HEK293 cells to obtain transformed cells. The culture solution of these transformed cells can be purified by chromatography. The method for making the bispecific antibodies are already well known, and JP2019022497, JP2017137329, and JP2015110628 can be referred to.

The class of the antibody is not particularly limited, and antibodies having any isotypes, such as IgG, IgM, IgA, IgD, or IgE, are included. In consideration of, for example, the ease of purification, IgG is preferred.

Examples of domain structures include low molecular weight antibodies, such as antibody fragments, and modified antibodies. Specific examples of the antibody fragments include, but are not limited to, Fab, Fab', F(ab')₂, Fv, scFv, and diabodies. Examples described later show, but are not limited to, H-chain constant region of human IgG with amino acid mutations at 228, 233-238, 265, 268, 309, 318, 328-331 which are believed to suppress binding affinity to the Fc receptor and amino acid mutations of 349, 354, 366, 368, 407 for hetero H chain.

Among the antibodies of the present invention, the antibody which binds to the polypeptide (i) or (ii) may be any antibody which binds to these polypeptides. On the other hand, the antibody used for prevention and/or treatment of an MPN is preferably an antibody which binds to the polypeptide (i) or (ii) and has a cytotoxic activity.

The antibody which binds to the cleaved mutant CALR protein may be any antibody which binds to a polypeptide chain having a sequence of SEQ ID NO: 2 or 3, and is preferably an antibody that competes with at least one of the antibodies or functional fragments thereof for binding to an amino acid sequence portion of SEQ ID NO: 2 or 3 in the mutant CALR protein.

Examples of the component (B) include at least one drug selected from the group consisting of an alkylating agent, a platinum preparation, an antimetabolite, a ribonucleotide reductase inhibitor, a nucleotide analog, a topoisomerase inhibitor, a microtubule polymerization inhibitor, a Bcl-2 inhibitor, an antitumor antibiotic, an interferon, a cytokine preparation, a molecular targeting drug, a nucleic acid synthesis inhibitor, a JAK inhibitor, and a cancer immunotherapeutic agent, among which at least one drug selected from the group consisting of a JAK inhibitor, an antimetabolite (including a ribonucleotide reductase inhibitor), a nucleic acid synthesis inhibitor, an interferon, a cytokine preparation, a molecular targeting drug, and a cancer immunotherapeutic agent is preferred.

Examples of the alkylating agent include cyclophosphamide, ifosfamide, dacarbazine, temozolomide, nimustine, busulfan, and ranimustine. Examples of the platinum preparation include cisplatin, carboplatin, and oxaliplatin. Examples of the antimetabolite, the ribonucleotide reductase inhibitor, and the nucleotide analog include hydroxyurea, methotrexate, 5-FU, tegafur, 6-mercaptopurine, capecitabine, and pentostatin. Examples of the topoisomerase inhibitor include doxorubicin, daunorubicin, epirubicin, idarubicin, mitoxantrone, pirarubicin, amrubicin, etoposide, and irinotecan. Examples of the Bcl-2 inhibitor include venetoclax. Examples of the antitumor antibiotic include mitomycin C, actinomycin D, bleomycin, peplomycin, and zinostatin stimalamer. Examples of the microtubule polymerization inhibitor include paclitaxel and docetaxel. Examples of the JAK inhibitor include tofacitinib, baricitinib, peficitinib, upadacitinib, filgotinib, abrocitinib, and ruxolitinib. Examples of the interferon include interferon-α, interferon-β, and interferon-γ. Examples of the cytokine preparation include interleukin 2. Examples of the nucleic acid synthesis inhibitor include azathioprine, mizoribine, and cyclophosphamide. Examples of the molecular targeting drug and the cancer immunotherapeutic agent include antibody pharmaceuticals, such as anti-EGFR antibodies, anti-HER2 antibodies, anti-VEGF antibodies, anti-VEGFR antibodies, anti-PD-1 antibodies, anti-PD-L1 antibodies, and anti-CTLA-4 antibodies; EGFR agonists, such as gefitinib, erlotinib, afatinib, osimertinib, and dacomitinib; HER2 agonists such as lapatinib; mTOR agonists, such as everolimus, temsirolimus, and sirolimus; cancer immunocheckpoint inhibitors, such as anti-PD-1 antibodies, anti-PD-L1 antibodies, anti-CTLA-4 antibodies, and CTLA-4-Ig (cancer immunocheckpoint inhibitors are also classified as molecular targeting drugs).

Here, examples of the anti-PD-1 antibodies include nivolumab and pembrolizumab. Examples of the anti-PD-L1 antibodies include atezolizumab, durvalumab, and avelumab. Examples of the anti-CTLA-4 antibodies include ipilimumab and tremelimumab. Examples of the CTLA-4-Ig include abatacept.

Among these components (B), at least one drug selected from the group consisting of ruxolitinib, hydroxyurea, interferon α, and a cancer immunocheckpoint inhibitor is particularly preferred.

As shown in Examples described later, the combination of the component (A) and the component (B) (when used in combination) enhances antitumor effects on various cancers and is useful in a pharmaceutical for preventing and/or treating a cancer.

The component (A) is also useful as an antitumor effect enhancer of the component (B). In addition, the component (B) is useful as an antitumor effect enhancer of the component (A).

The form of the combination pharmaceutical of the present invention is not particularly limited, and specific examples thereof include the following forms (i) and (ii).
(i) A form of a single formulation (compounding agent) containing both components (A) and (B).
(ii) A form for separately administering a formulation containing the component (A) and a formulation containing the component (B).

In the case of the form according to (ii), each formulation may be administered simultaneously or separately at an appropriate time interval, and an appropriate dosage regimen can be adopted so that desired preventive and/or therapeutic effects on cancer are achieved. In the form for separately administering a formulation containing the component (A) and a formulation containing the component (B), it is also possible to provide a kit formulation containing both formulations in a single package.

Specific examples of the cancer, which are the subject of the present invention, include lymphoma, leukemia, multiple myeloma, a myeloproliferative neoplasm (MPN), head and neck cancer, gastrointestinal cancer (esophageal cancer, gastric cancer, duodenal cancer, and the like), liver cancer, biliary tract cancer (gallbladder/bile duct cancer, and the like), pancreatic cancer, small intestine cancer, large intestine cancer (colorectal cancer, colon cancer, rectal cancer, and the like), gastrointestinal stromal tumor, lung cancer (non-small cell lung cancer, small-cell lung cancer), breast cancer, ovarian cancer, uterine cancer (cervical cancer, endometrial cancer, and the like), kidney cancer, bladder cancer, prostate cancer, and skin cancer. Here, the cancer includes not only the primary lesion but also cancer that has metastasized to other organs (liver, and the like).

The pharmaceutical of the present invention is more useful for lymphoma, leukemia, multiple myeloma, a myeloproliferative neoplasm (MPN), gastrointestinal cancer (esophageal cancer, gastric cancer, duodenal cancer, and the like), liver cancer, biliary tract cancer (gallbladder/bile duct cancer, and the like), pancreatic cancer, small intestine cancer, large intestine cancer (colorectal cancer, colon cancer, rectal cancer, and the like), gastrointestinal stromal tumor, lung cancer (non-small cell lung cancer, small-cell lung cancer, and the like), kidney cancer, and the like, is further useful for lymphoma, leukemia, multiple myeloma, a myeloproliferative neoplasm (MPN), and the like, and is even more useful for cancer in which mutant CALR is detected, especially MPN.

As described above, the pharmaceutical of the present invention may be in the form of a composition containing the component (A), a composition containing the component (B), or a composition containing the component (A) and the component (B). These compositions can be produced by formulating the components (A) and/or (B) together with a pharmaceutically acceptable carrier through, for example, mixing, dissolving, emulsifying, encapsulating, or lyophilizing.

Suitable formulations for oral administration are, for example, liquids prepared by dissolving an effective amount of the component (A) and/or the component (B) in diluents such as water and physiological saline; capsules, granules, powders, or tablets containing an effective amount of the component (A) and/or the component (B) as solids or granules; suspensions prepared by suspending an effective amount of the component (A) and/or the component (B) in an appropriate dispersion medium, and emulsions prepared by dispersing and emulsifying, in an appropriate dispersion medium, a solution prepared by dissolving an effective amount of the component (A) and/or the component (B).

For parenteral administration, the component (A) and/or the component (B) can be formulated into dosage forms such as a solution for injection, a suspension, an emulsion, a cream, an ointment, an inhalant, and a suppository together with pharmaceutically acceptable solvents, excipients, binders, stabilizers, dispersants, and the like. In the prescription for injection, the component (A) and/or the component (B) can be dissolved in an aqueous solution, preferably a physiologically compatible buffer such as Hanks' solution, Ringer's solution, or physiological saline buffer. Furthermore, the pharmaceutical of the present invention can be in forms such as a suspension, a solution, or an emulsion in an oily or aqueous vehicle. Alternatively, the component (A) and/or the component (B) may be produced in a form of powder, and an aqueous solution or a suspension may be prepared using sterile water or the like before use. For administration by inhalation, the component (A) and/or the component (B) is powdered, and a powder mixture can be formed with an appropriate base such as lactose or starch. A suppository prescription can be produced by mixing the component (A) and/or the component (B) with a commonly used suppository base such as cocoa butter. Furthermore, the pharmaceutical of the present invention can be prescribed as a sustained release formulation by encapsulating the agent in a polymer matrix or the like.

The component (B) can be administered according to its dosage and administration when already commercially available. The component (A) is preferably administered in an amount of 0.3 mg or more per dose, more preferably from 0.3 mg to 300 mg per dose, and further preferably from 3 mg to 300 mg per dose.

### Examples

Next, the present invention will be described in more detail with reference to Examples, but the present invention is not limited to these Examples at all.

### [Test Example 1] Making of antibodies which recognize mutant CALR protein containing cleaved type

A mouse monoclonal antibody clone CAL2 (Cat# DIA-CAL) manufactured by Dianova or a peptide containing cysteine added to the C-terminus or N-terminus of the amino acid sequences contained in SEQ ID NO: 2 to 5 was synthesized and conjugated to a carrier protein, keyhole limpet hemocyanin (KLH), then immunized to 8-week-old female WKY/Izm rats. After booster immunization, lymphocytes were collected. The lymphocytes were cellfused with mouse myeloma SP2 cells by a PEG method, then cultured in a selective medium to obtain hybridomas. The specificity of antibodies in the culture supernatant was screened by an ELISA method using the immunized peptide or purified protein to obtain hybridomas (clones B3, C6, and G1) producing antibodies which bind to mutant CALR proteins containing a cleaved type. Antibodies produced by these hybridomas (clone B3 antibody, C6 antibody, and G1 antibody) were purified and used in Tests.

### [Test Example 2] Making of mutant CALR expressing cells

Human megakaryoblastic leukemia cell line UT-7/TPO cells were transfected with a vector expressing a Del 52 or Ins 5 type mutant CALR protein and a vector used for transfection as a control. The resulting UT-7/TPO CALR Del 52 cells and UT-7/TPO CALR Ins 5 cells neoplastically proliferating, and UT-7/TPO vec cells were each seeded at a density of 6.0 × 10⁵ cells/mL in Opti-MEM medium (Thermo Fisher Scientific K.K.) and cultured in the presence of 5% CO₂ at 37°C for 32 hours. On this occasion, UT-7/TPO vec cells were cultured in a medium containing 10 ng/mL TPO.

### [Test Example 3] Verification of recognition of mutant CALR proteins

UT-7/TPO cells expressing a Del 52 type or Ins 5 type mutant CALR having a FLAG-tag inserted downstream of the signal sequence at the N-terminus were cultured, and culture supernatants containing the secreted mutant CALR proteins were prepared by centrifugation (1,600 g × 5 mins, 4°C). The culture supernatants were heat-treated in the presence of SDS and a reducing agent, then resolved on a gel by SDS polyacrylamide electrophoresis, electrically transferred on a polyvinylidene difluoride (PVDF) membrane, and reacted with a TBS-T solution containing 5% skim milk at room temperature for 1 hour, followed by reaction with a 5% BSA/TBS-T solution containing a rat clone B3, C6, or G1 antibody or mouse anti-DYKDDDDK tag antibody (FUJIFILM Wako Pure Chemical Corporation) at 4°C overnight. After washing with a TBS-T solution, the reaction products were each reacted with a 5% skim milk/TBS-T solution containing a peroxidase-labeled goat anti-rat IgG antibody (Jackson Immuno Research Inc.) or a peroxidase-labeled goat anti-mouse IgG antibody (Jackson Immuno Research Inc.) at room temperature for 1 hour. After washing with a TBS-T solution, the PVDF membranes were reacted with a peroxidase luminescence reagent. The resulting signals were detected using a FUSION image pickup apparatus.

As a result, it was verified that all of the clone B3, C6, and G1 antibodies recognize the full-length and cleaved mutant CALR proteins, while CAL2 recognizes the full-length CALR protein and the C-terminal sequence from the recognition sequences of the B3, C6, and G1 antibodies.

### [Test Example 4] Determination of sequence information of antibody of present invention

To obtain the sequence information of heavy-chain and light-chain antibodies of the present invention, mRNAs were prepared from cells producing the antibodies using a PureLinc RNA Mini kit (Thermo Fisher Scientific K.K.). After cDNAs were synthesized using the resulting mRNAs as templates and reverse transcription primers specific to the heavy chain or the light chain by rapid amplification of cDNA ends method, the cDNAs were cloned into plasmids. The resulting plasmids were analyzed by sanger sequencing to determine the full-length sequences of the cDNAs of the heavy chain and light chain.

### [Example 1] Preparation of bispecific antibodies Material and test method

Bispecific antibody expression vectors were constructed using DNA encoding bispecific antibodies based on sequence information of clone B3, C6, and G1 antibodies and anti-CD3 antibodies (Table 1) and expression vectors (pcDNA3.4, Thermo Fisher Scientific K.K.). The expression vectors were transfected into CHO cells or HEK293 cells by an ExpiFectamine CHO Transfection Kit (Thermo Fisher Scientific K.K.) or an ExpiFectamine 293 Transfection Kit (Thermo Fisher Scientific K.K.), the transformed cells were cultured, then the cells were removed by centrifugation and filtration to collect the culture solution. Purification of the antibodies was performed by a combination of affinity chromatography using nickel-conjugated agarose and gel filtration chromatography, a combination of ProteinA affinity chromatography and gel filtration chromatography, or a combination of affinity chromatography using a CaptureSelect kappa XL resin (Thermo Fisher Scientific K.K.) and gel filtration chromatography. In the same manner as above, based on sequence information described in Patent Literature 4 and Patent Literature 2, bispecific antibodies (Antibody 32: SEQ ID NO: 114, and SEQ ID NO: 115, antibody 33: SEQ ID NO: 116, and SEQ ID NO: 117, antibody 34: SEQ ID NO: 118, and SEQ ID NO: 119) and B3 chimeric antibodies (SEQ ID NO: 52 and SEQ ID NO: 64) were prepared.

### [Example 2] Verification of recognition of mutant CALR protein on cell surface by flow cytometry analysis Material and test method

UT-7/TPO vec cells transfected with only the vector used for transfection as a control and mutant CALR-expressing UT-7/TPO CALR Ins 5 cells and UT-7/TPO CALR Del 52 cells were cultured using an IMDM medium containing 10% fetal bovine inactivated serum (FBS) in the presence of 5% CO₂ at 37°C. The 5 × 10⁴ cells in the proliferative stage were centrifuged (400 g × 5 min, 4°C), and then various antibodies were prepared in an FBS/PBS solution at a concentration of 5 µg/mL and reacted on ice for 30 minutes. After the reaction, the cells were washed twice using a MACS buffer, and an anti-human Fc antibody was prepared as a secondary antibody in an FBS/PBS solution at a concentration of 2.5 µg/mL and reacted on ice for 30 minutes.

After completion of the reaction, the cells were washed twice by adding the MACS buffer and performing centrifugation (400 g × 5 min, 4°C), and discarding the supernatant. Signals were quantitatively measured by flow cytometry analysis using BD LSRFortessa X-20 (BD Biosciences).

[a] UT-7/TPO vec cells, [b] UT-7/TPO CALR Ins 5 cells, and [c] UT-7/TPO CALR Del 52 cells were analyzed by flow cytometry using representative anti-cleaved mutant CALR-CD3 bispecific antibodies made using a VH chain of SEQ ID NO: 6, a VL chain of SEQ ID NO: 3, a VH chain of SEQ ID NO: 8, and a VL chain of SEQ ID NO: 9.

### [Example 3] Verification of recognition of CD3 antigen on cell surface by flow cytometry analysis

### Material and test method

Peripheral blood mononuclear cells (PBMC) isolated from healthy human donors were cultured for two days in plates coated with an anti-CD3 antibody (Takara Bio Inc.) from 4 to 24 hours, further cultured in an RPMI-1640 medium containing IL-2 and 10% FBS (Sigma-Aldrich) from 10 to 21 days to induce T-LAK cells. The 5 × 10⁵ T-LAK cells were centrifuged (400 g × 5 min, 4°C), and then anti-cleaved mutant CALR-CD3 bispecific antibodies made using a VH chain of SEQ ID NO: 2, a VL chain of SEQ ID NO: 3, a VH chain of SEQ ID NO: 8, and a VL chain of SEQ ID NO: 9 in an FBS/PBS solution were prepared at a concentration of 5 µg/mL and reacted on ice for 30 minutes.

After the reaction, the cells were washed twice using a MACS buffer, and an anti-human Fc antibody was prepared as a secondary antibody in an FBS/PBS solution at a concentration of 5 µg/mL and reacted on ice for 30 minutes. After completion of the reaction, the cells were washed twice by adding the MACS buffer, performing centrifugation (400 g × 5 min, 4°C), and discarding the supernatant. Signals were quantitatively measured by flow cytometry analysis using BD LSRFortessa X-20 (BD Bioscience).

As a result, it was verified that the bispecific antibodies specifically recognize CD3 antigens expressed on the T-LAK cell surface.

### [Example 4] Evaluation of in vitro cytotoxic activity

### (Material and test method)

UT-7/TPO/Ins 5-Luc cells as target cells were cultured with T cells in the presence of the bispecific antibodies and hydroxyurea, ruxolitinib, or interferon α, and then the viability of the target cells was determined using luciferase activity as an indicator to evaluate T cell-dependent cellular cytotoxicity (hereinafter referred to as TDCC).

The T cells were isolated and concentrated from healthy human blood using RosetteSep Human T Cell Enrichment Cocktail (STEMCELL technologies) or from healthy human peripheral blood mononuclear cells (PBMCs) using Pan T cell isolation kit (Miltenyi biotech).

In a 384 well plate, 20 µL of T cell suspension, 20 µL of target cells, 5 µL of anti-bispecific antibody diluent, and 5 µL of combined drug diluent were added to each well (final volume: 50 µL/well; T cells: 5 × 10⁴ cells/well; target cells: 5 × 10³ cells/well; E/T = 10) and cultured at 37°C in 5% CO₂ for 48 hours, followed by addition of 25 µL of Steady-Glo Luciferase Assay reagent to each well, to measure chemiluminescence of each well.

The cell viability of each well was calculated using the chemiluminescence value of a control well without bispecific antibody and combined drug as 100%.

The combination effect was evaluated using a combination index (CI). The CI was calculated using CompuSyn software (Combosyn, Inc.) based on the Chou-Talalay method (Chou T.C. & Talalay, P.: Adv.Enz.Regul.22: 27-55, 1984). A CI below 0.7 is determined to have a synergistic effect, and a CI below 0.3 is determined to have a strong synergistic effect. A CI of around 1 is determined to have an additive effect. Table 3 summarizes, as representative values of CI, the values of CI for the combination of the lowest concentration at a cytotoxic activity exceeding 85% with combination effects verified.

As shown in Figures 2-A to 4 and Table 3, the combination of the antibody of the present invention and the drug showed cytotoxic activity against UT-7/TPO CALR Ins 5 cells expressing mutant CALR.

**[Table 3]**

| Combination effect of antibody with drug | | |
|---|---|---|
| Combined drug | Antibody | CI (Cytotoxicity at level of 85% |
| Hydroxyurea | Antibody 1 | 0.21 |
| | Antibody 2 | 0.17 |
| | Antibody 3 | 0.13 |
| | Antibody 4 | 0.35 |
| | Antibody 5 | 0.29 |
| | Antibody 6 | 0.16 |
| | Antibody 7 | 0.42 |
| | Antibody 8 | 0.38 |
| | Antibody 9 | 0.50 |
| Ruxolitinib | Antibody 1 | 0.50 |
| | Antibody 7 | 0.50 |
| | Antibody 8 | 0.33 |
| | Antibody 9 | 0.55 |
| Interferon α | Antibody 1 | 0.05 |
| | Antibody 4 | 0.01 |
| | Antibody 5 | 0.11 |
| | Antibody 6 | 0.01 |
| | Antibody 8 | 0.21 |
| | Antibody 9 | 0.18 |

| | | |
|---|---|---|
| CI < 0.7 synergistic effect, CI≒ 1 additive effect | | |

## Claims

1. A pharmaceutical for preventing and/or treating a cancer, comprising (A) an antibody which specifically binds to a mutant calreticulin protein or a functional fragment thereof and (B) at least one drug selected from the group consisting of an alkylating agent, a platinum preparation, an antimetabolite, a ribonucleotide reductase inhibitor, a nucleotide analog, a topoisomerase inhibitor, a microtubule polymerization inhibitor, a Bcl-2 inhibitor, an antitumor antibiotic, an interferon, a cytokine preparation, a molecular targeting drug, a nucleic acid synthesis inhibitor, a JAK inhibitor, and a cancer immunotherapeutic agent in combination.

2. The pharmaceutical according to claim 1, wherein the component (A) is an antibody or a functional fragment thereof which does not bind to a wild-type calreticulin protein but has high affinity to the mutant calreticulin protein.

3. The pharmaceutical according to claim 1 or 2, wherein the component (A) is an antibody or a functional fragment thereof which binds to a mutant amino acid sequence of SEQ ID NO: 2 or 3 or a sequence having 80% or more homology to the mutant amino acid sequence.

4. The pharmaceutical according to any one of claims 1 to 3, wherein the component (A) is at least one antibody or a functional fragment thereof selected from the group consisting of a human antibody, a humanized antibody, a bispecific antibody, a multispecific antibody, a chimeric antibody of an animal-derived antibody and a human antibody, Fab, Fab', F(ab')₂, a single-chain antibody (scFv), and a diabody.

5. The pharmaceutical according to any one of claims 1 to 4, wherein the component (B) is at least one drug selected from the group consisting of a JAK inhibitor, a nucleic acid synthesis inhibitor, an interferon, a cytokine preparation, a molecular targeting drug, and a cancer immunotherapeutic agent.

6. The pharmaceutical according to any one of claims 1 to 5, wherein the component (B) is at least one drug selected from the group consisting of ruxolitinib, hydroxyurea, interferon α, and an immunocheckpoint inhibitor.

7. The pharmaceutical according to any one of claims 1 to 6, wherein the cancer is selected from the group consisting of lymphoma, leukemia, multiple myeloma, a myeloproliferative neoplasm (MPN), gastrointestinal cancer (such as esophageal cancer, gastric cancer, and duodenal cancer), liver cancer, biliary tract cancer (such as gallbladder/bile duct cancer), pancreatic cancer, small intestine cancer, large intestine cancer (such as colorectal cancer, colon cancer, and rectal cancer), gastrointestinal stromal tumor, lung cancer (such as non-small cell lung cancer and small-cell lung cancer), and kidney cancer.

8. Use of a combination of (A) an antibody which specifically binds to a mutant calreticulin protein or a functional fragment thereof and (B) at least one drug selected from the group consisting of an alkylating agent, a platinum preparation, an antimetabolite, a ribonucleotide reductase inhibitor, a nucleotide analog, a topoisomerase inhibitor, a microtubule polymerization inhibitor, a Bcl-2 inhibitor, an antitumor antibiotic, an interferon, a cytokine preparation, a molecular targeting drug, a nucleic acid synthesis inhibitor, a JAK inhibitor, and a cancer immunotherapeutic agent, for producing a pharmaceutical for preventing and/or treating a cancer.

9. A combination of (A) an antibody which specifically binds to a mutant calreticulin protein or a functional fragment thereof and (B) at least one drug selected from the group consisting of an alkylating agent, a platinum preparation, an antimetabolite, a ribonucleotide reductase inhibitor, a nucleotide analog, a topoisomerase inhibitor, a microtubule polymerization inhibitor, a Bcl-2 inhibitor, an antitumor antibiotic, an interferon, a cytokine preparation, a molecular targeting drug, a nucleic acid synthesis inhibitor, a JAK inhibitor, and a cancer immunotherapeutic agent, for use in preventing and/or treating a cancer.

10. A method for preventing and/or treating a cancer, comprising administering a combination of (A) an antibody which specifically binds to a mutant calreticulin protein or a functional fragment thereof in combination and (B) at least one drug selected from the group consisting of an alkylating agent, a platinum preparation, an antimetabolite, a ribonucleotide reductase inhibitor, a nucleotide analog, a topoisomerase inhibitor, a microtubule polymerization inhibitor, a Bcl-2 inhibitor, an antitumor antibiotic, an interferon, a cytokine preparation, a molecular targeting drug, a nucleic acid synthesis inhibitor, a JAK inhibitor, and a cancer immunotherapeutic agent in combination.
